(19) Europäisches Patentamt
European Patent Office
Office européen des brevets

(11) **EP 4 044 904 B1**

(12) **EUROPEAN PATENT SPECIFICATION**

(45) Date of publication and mention
of the grant of the patent:
**29.05.2024 Bulletin 2024/22**

(21) Application number: **20797863.6**

(22) Date of filing: **15.10.2020**

(51) International Patent Classification (IPC):
*A61B 5/00* *(2006.01)*     *A61B 5/107* *(2006.01)*

(52) Cooperative Patent Classification (CPC):
**A61B 5/6824; A61B 5/1071; A61B 5/6828;
A61B 5/6829;** A61B 5/6832; A61B 5/7246;
A61B 2505/09

(86) International application number:
**PCT/IB2020/059717**

(87) International publication number:
**WO 2021/074852 (22.04.2021 Gazette 2021/16)**

(54) **SENSOR DETERMINATION**

SENSORBESTIMMUNG

DÉTERMINATION DE CAPTEUR

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **18.10.2019 GB 201915139**

(43) Date of publication of application:
**24.08.2022 Bulletin 2022/34**

(73) Proprietor: **McLaren Applied Limited
Woking
Surrey GU21 6JD (GB)**

(72) Inventors:
• **GASKELL, Matthew Alastair**
**London N19 4NN (GB)**
• **REDDALL, Nicholas Henry**
**London SW18 5UF (GB)**

(74) Representative: **Röthinger, Rainer
Wuesthoff & Wuesthoff
Patentanwälte und Rechtsanwalt PartG mbB
Schweigerstrasse 2
81541 München (DE)**

(56) References cited:
WO-A1-2018/092944     WO-A1-2019/112158
US-A1- 2017 238 849     US-A1- 2019 038 225

## Description

FIELD

This invention relates to a method for determining an unworn state of a system for providing information about a joint.

BACKGROUND

There is a growing popularity for devices that measure movement. These sensing devices can be in the form of wearable devices that measure movement of a user, a smartphone that is carried by the user to measure movement of the user or moveable devices that can generally sense movement, for instance video game controllers or sensors attached to industrial equipment. In particular, wearable devices can be utilised to track motion of a human or other animal and, in particular can be used to monitor the motion of a specific joint.

These sensing devices may include a satellite positioning sensor which can sense the location of the device, and one or more motion sensors which sense motion and/or orientation of the device. These motion sensors may include one or more of an accelerometer, a gyroscope, a magnetometer, a compass and a barometer. Measurements taken by the sensors can be used to provide information about the joint.

When using a wearable device, it may be necessary for the device to be used for extended periods of time such as a month or more so as to build up data which changes only slowly over time. This means that any sensing device which is used will likely need to be removed for any number of reasons including, but not limited to, the need to recharge a power supply on the device, the desire to clean the sensor so as to remove a build-up of dirt and grime or spillages thereon, or to wash the part of the person or animal on which the sensor is mounted. Such a sensing device could also fall off. If measurements taken by the device when it has been removed or otherwise dislodged from its intended location are included when providing the information, this could be misleading as regards monitoring the joint in question.

Thus, it would be desirable for there to be improvements in how wearable sensors are operated.

US 2019/038225 A1 discloses a wearable apparatus for monitoring activity of a body joint such as knee or elbow for monitoring rehabilitation and recovery after surgical procedures and after injury to the joint is disclosed. The wearable apparatus includes a wearable harness in which a first portion of the wearable harness includes a proximal strap to be positioned proximal to the body joint and in which a second portion of the wearable harness includes a distal strap to be positioned distal to the body joint, in which the wearable harness is positionable upon a patient's body at the body joint without occluding a surgical site of the patient. Such a wearable harness further includes a connecting section having a first end connected with the proximal strap and a second end connected with the distal strap and a first buckle housing (smart buckle) secured to the proximal strap of the wearable harness and a second buckle housing secured to the distal strap of the wearable harness, in which the proximal and distal straps maintain the first and the second buckles in a consistent position relative to the axis of an underlying bone such as a thigh bone, calf bone, femur, or tibia of a patient. The wearable harness further includes integrated circuitry including at least a magnetometer and accelerometer within the first buckle and an accelerometer within the second buckle. A microprocessor of the smart buckle periodically reads a switch position on the buckles, reads the magnetometer and accelerometer data and processes the data to determine (i) an angle for the sample period, and periodically classify an activity. Such data is relayed to a cloud computing environment for further analysis and for access by clinicians and patients.

WO 2019/112158 A1 discloses an inertial sensor error correction device which comprises: a first inertial sensor unit which is disposed on one side of a first support unit and measures, in real time, a first measured value, namely the angle of the first support unit; a second inertial sensor unit which is disposed on a second support unit and one side end of which is connected to another side end of the first support unit via a joint unit, so as to measure, in real time, a second measured value, namely the angle of the second support unit; a joint sensor which is mounted on the joint unit and measures, in real time, a third measured value, namely the joint angle between the first support unit and the second support unit; and a correction unit which corrects at least one out of the first inertial sensor unit and the second inertial sensor unit based on the third measured value, measured at the joint sensor.

SUMMARY

The present invention is defined in the independent claims. Advantageous embodiments are set forth in the dependent claims and hereinbelow.

According to a first aspect of the present disclosure, there is provided a method according to claim 1.

In some variations, obtaining one or more measurements from each sensor may be performed multiple times in a time period and the calculating a relative angle and the calculating a joint angle may be performed using measurements obtained at at least some of the multiple times. The method may include the further step of, following a determination that the sensor system is in an unworn state, adopting a different procedure with regard to calculating the joint angle at subsequent times. The different procedure can comprise any one or more of: not calculating the joint angle at subsequent times; differently processing the calculated joint angles at subsequent times from those calculated at times prior to the determination; not storing the joint angles calculated at subsequent times.

The method may further comprise correlating the joint angles and relative angles calculated at multiple times to provide information about the joint over the time period, and adopting a different procedure may comprise omitting any data calculated using measurements made at subsequent times from the information. The information may comprise any one or more of: joint angle variation during the time period; duration of time in which the joint is active; duration of time in which the joint is bearing a load; length of the time period; and if the joint is a knee, step count over the time period.

Adopting a different procedure can comprise performing calculations to provide different information from the information about the joint, wherein the different information includes any one or more of: unworn time; and if the first sensor is determined to be charging, charging time.

An unworn state may include any one or more of: the first sensor having been at least partially removed from its mounted position; the first sensor falling off its mounted position at least to some extent; the first sensor being switched on but not mounted on the first body part; the second sensor being switched on but not mounted on the second body part.

The determining if the first sensor is mounted on the first body part may comprise calculating a value of a function of the calculated angles. The method may further comprise processing multiple calculated values of the function over a time period to determine a moving average value for the function and wherein the determining may further comprise comparing the determined moving average value to a threshold value. The function can comprise a function of a joint angle penalty and a relative angle penalty. In some implementations, if the joint angle is within a feasible range for the joint, the joint angle penalty may be zero and if the joint angle is not within a feasible range for the joint, the joint angle penalty may have a value which depends on how far outside the feasible range the joint angle is. In some implementations, if the relative angle is less than or equal to a threshold relative value, the relative angle penalty may be zero and if the relative angle penalty is greater than the threshold relative value, the relative angle penalty may depend on how much greater the relative angle is than the threshold relative value.

The relative angle may be a difference in tilt angle of the two body parts, where tilting occurs about an axis perpendicular or substantially perpendicular to the plane in which the joint angle is defined.

The joint angle can be defined in an x-z plane and the tilt angle can be defined in the x-y plane and may be non-zero if the first and second body parts undergo a relative roll about the x-axis.

The method may further comprise, prior to the obtaining, determining whether the first and second sensors are mounted on correct respective first and second body parts and/or in substantially a predetermined orientation relative to the correct respective first and second body parts, and if one or both sensors is determined to be mounted on the wrong body part or in a different orientation from the predetermined orientation, adjusting the calculating steps to take account of an actual determined mounting location and/or orientation.

The method may further comprise, prior to the obtaining, calibrating the first and second sensors with respect to a predetermined orientation relative to the respective first and second body parts.

The joint may be a knee or an elbow. The joint could be other joints such as a shoulder or ankle joint.

Any of the above methods can be used in any feasible combination

According to a second aspect of the invention, there is provided a system according to claim 12.

The first and second sensor units may be configured to take measurements multiple times in a time period and the calculation unit may be configured to calculate a relative angle and a joint angle using measurements obtained at each of at least some of the multiple times. The calculation unit may be further configured to, following a determination that the system is in an unworn state, adopt a different procedure with regard to calculating the joint angle at subsequent times. The calculation unit may be further configured to correlate the joint angles and relative angles calculated at multiple times to provide information about the joint over the time period, and in some implementations, adopting a different procedure may comprise omitting any data relating to measurements made at subsequent times from the information.

The second, master sensor unit may be configured to provide the information to a mobile device for viewing by a user. The information may be for use in assessing health and/or rehabilitation of the joint.

In some examples, the first and second sensor units may be generally planar having front and back faces and may be configured for their back faces to be mounted on the first and second body parts at a side of the joint substantially parallel to the plane of normal bending of the joint.

DRAWINGS

The present invention will now be described by way of example with reference to the accompanying drawings, in which:

    Figure 1 shows a diagram of a joint;

    Figure 2 shows a direction reference frame for a joint;

    Figure 3 shows a pair of sensors fitted either side of a joint in accordance with some implementations;

    Figure 4 shows a schematic diagram of a system for monitoring a joint;

Figure 5 is a diagram showing some of the functions of a system for monitoring a joint;

Figure 6 shows one of the sensors of figure 3 being removed;

Figure 7a shows a graph of knee angle;

Figure 7b shows a graph of a roll angle; and

Figure 8 shows a method in accordance with some implementations.

In the figures, like reference numerals indicate like parts.

DETAILED DESCRIPTION

The current subject-matter relates to a method for determining if a body sensor is in an unworn state and a system for implementing the method. The method is particularly directed to a system in which a pair of sensors is mounted either side of a joint, where the sensors are being used to monitor the angle of the joint over time. For example, if a person has injured the joint or had surgery on the joint, they may not be able to bend it through the full range of movement of a healthy joint. By monitoring the movement of the joint over time, a picture can be built up of the person's activity level and whether the range of movement of the joint is improving.

If this information is to be useful, it is preferable for it to include only data obtained when the sensors are mounted in their intended position on the person. If data obtained when this is not the case is included in the information, this could skew the information such that it becomes less useful in monitoring the joint. Implementations of the present invention aim to avoid such skewing of the information.

The following describes specific examples of the use of sensors in relation to a knee joint on a human. However, the underlying principles are applicable to many different joints such as the ankle, elbow or wrist, and could also be applied to joints associated with other animals.

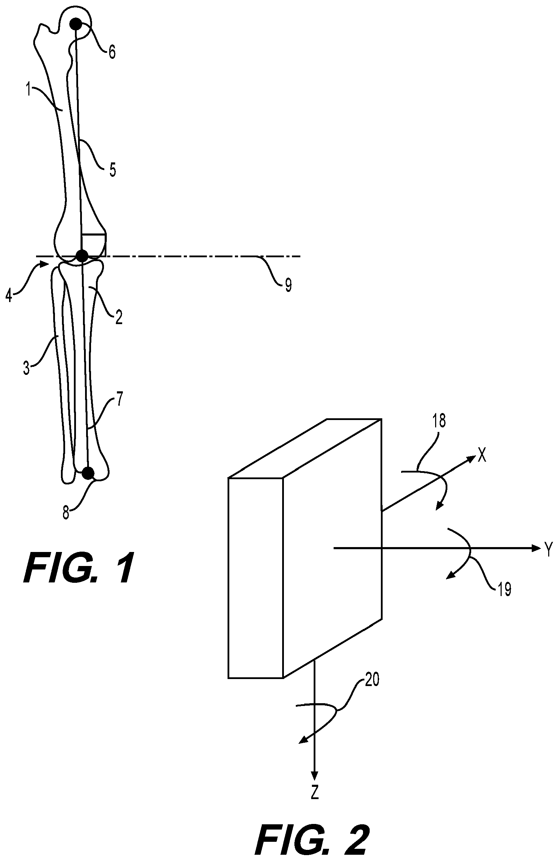

Figs. 1 and 2 are provided to allow a simple explanation of certain terms that are used within this specification. Fig. 1 illustrates a standard leg having a femur 1, a tibia 2 and a fibula 3. These are joined at a knee joint 4. The femur 1 defines a femoral mechanical axis 5 extending from the knee to a ball joint 6 which forms part of the person's hip. A tibial mechanical axis 7 extends from the knee 4 to the lower end 8 of the tibia 2 itself. The femur and the lower leg (made up of the tibia 2 and fibula 3) can pivot relative to each other about a knee joint axis 9. The femur and the lower leg thus define a plane in which the respective mechanical axes pivot relative to each other. Thus, each mechanical axis will substantially align with the respective part of the leg, such that the knee joint axis 9 is perpendicular to the plane in which the axes pivot. Thus the knee angle is thus typically the angle between the two mechanical axes.

This is an idealised situation, which forms the basic geometry considered by the present invention. It is possible to apply one or more compensation schemes to deal with any misalignment between the axes and the respective part of the leg.

Fig. 2 helps to define the coordinate system associated with the knee joint, as well as how the terms pitch and roll apply to the knee. The convention when discussing the knee joint is that, when a person is standing upright, the x-axis points forward i.e. away from the knee in the direction of walking, parallel to the ground, the y-axis points to the person's right, and the z-axis points downwards towards the ground. This convention applies to both left and right legs, i.e. the positive y-axis is always to the right hand side of the knee irrespective of the leg. The y-axis is therefore analogous to the knee joint axis 9.

The orientation of any sensors associated with the knee typically has two components. A rotation of the sensor about the x-axis is a roll motion, identified by an arrow 18, and defines a roll angle. A rotation of the sensor about the y-axis is a pitch motion, identified by an arrow 19, and defines a pitch angle. A third component, namely rotation of the sensor about the z-axis, would be a yaw motion, identified by an arrow 20, and would define a yaw angle.

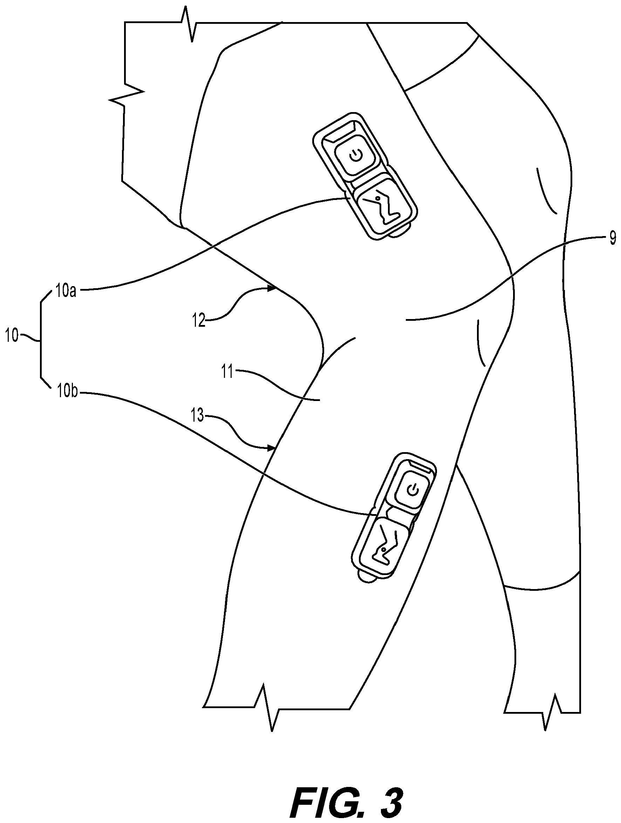

Fig. 3 illustrates a pair of sensors 10 attached to a leg 11. Each sensor contains one or more motion sensing devices which permit either (i) the pitch and/or roll of the individual sensor to be determined or (ii) the relative pitch and/or roll between the sensors to be determined. These motion sensing devices could be any suitable devices such as, but not limited to, an accelerometer, gyroscope, or a pair of strain gauges.

An upper sensor 10a is placed on the thigh 12 and a lower sensor 10b is placed on the calf 13. The purpose of the sensors is to monitor the flex of the knee at the knee joint, i.e. a pitch angle about the y-axis/knee joint axis 9. If the two sensors 10a, 10b could be aligned such that the z-axis of the sensor was parallel to the respective mechanical axis of the leg, and the sensor y-axis was parallel with the knee joint axis 9, the calculation of the knee angle would be a simple subtraction of the calf pitch angle from the thigh pitch angle. In practice, there may be a misalignment with the femoral and tibial mechanical axes which needs to be corrected for in order to obtain an accurate knee angle measurement.

In the example of the patient having had a total knee replacement or indeed any other knee surgery or knee complaint which results in limited movement of the knee, it can be helpful for a healthcare professional, or even the patient themselves, to monitor the knee angle over a lengthy period of time such as weeks or even months. Thus, a further problem can arise as the sensors will need to be removed periodically for numerous reasons including but not limited to cleaning of the sensor, recharging the sensors, increasing the comfort of the patient at night or cleaning of the patient in the sensor locations. When the sensors are removed or indeed if they fall off or become dislodged from the position in which knee angle has been being monitored, the sensors are said to be in an unworn state. It would be beneficial to know if one or both of the sensors has entered an unworn state, because this indicates that the sensor system of which they form part is in an unworn state. Thus this can be taken account of when presenting data for the monitoring. Systems and methods for detecting an unworn state are described in the following.

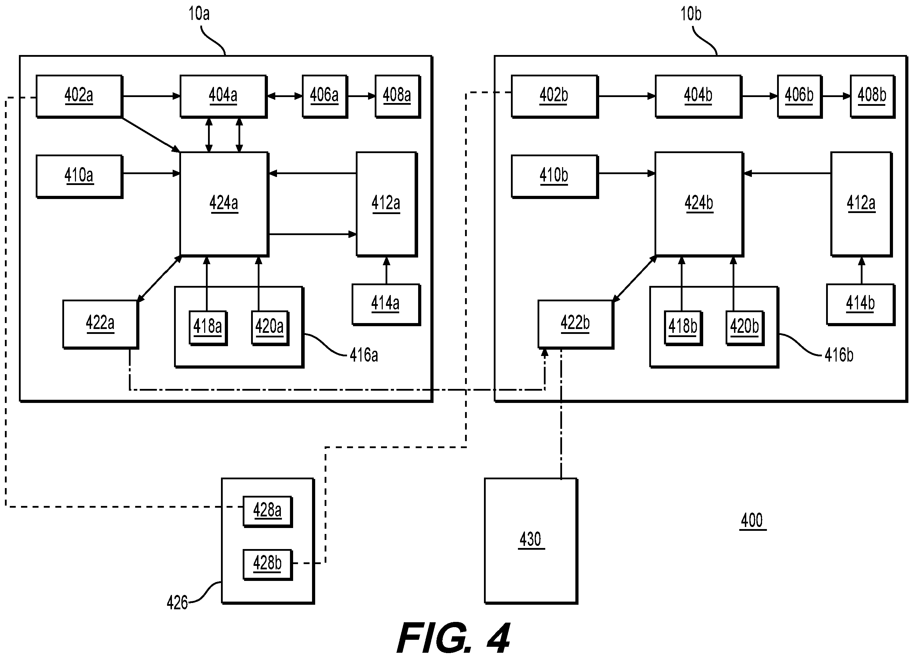

Fig. 4 illustrates schematically a sensor system 400 which includes the knee sensors 10, some components of the knee sensors 10 and operational connections to and from the sensors, and components thereof. It will be appreciated that the sensors 10 may each form part of a sensor unit configured to be mounted on a body part on a side of a joint as described with reference to Fig. 3. Thus each sensor 10 may form part of or be attached to some sort of patch that can be applied to skin or may be used in conjunction with an adhesive or other attachment means to enable mounting of the sensor to a body part.

The two sensors 10a, 10b comprise identical or similar components, which in each case are denoted by a same reference numeral and an a or b respectively. These components are: a power receiver/voltage regulator 402 connected to a cell charger and monitor 404, connected to a cell 406 (e.g. a Lithium-ion cell or other suitable energy storage device), connected to power supplies and support circuitry 408; a temperature sensor 410, which may in practice include any of multiple temperature sensors e.g. an external temperature sensor to sense temperature of skin to which the sensor unit is mounted, one or more internal temperature sensors to sense temperature of one or more components of the sensor itself; a power control/push switch logic unit 412 connected to an on/off push switch 414; an Inertial Measurement Unit (IMU) 416 which includes an accelerometer 418 and a gyroscope 420; and a Bluetooth Low Energy (BLE) module 422. All of the above-discussed components are connected to a microcontroller 424. The aforementioned on-sensor connections are shown with solid line arrows. It will be appreciated that further components may be present in the sensors 10 such as a magnetometer (to determine absolute movements and orientations of the patient), visual indicators of status etc., but these are omitted for clarity.

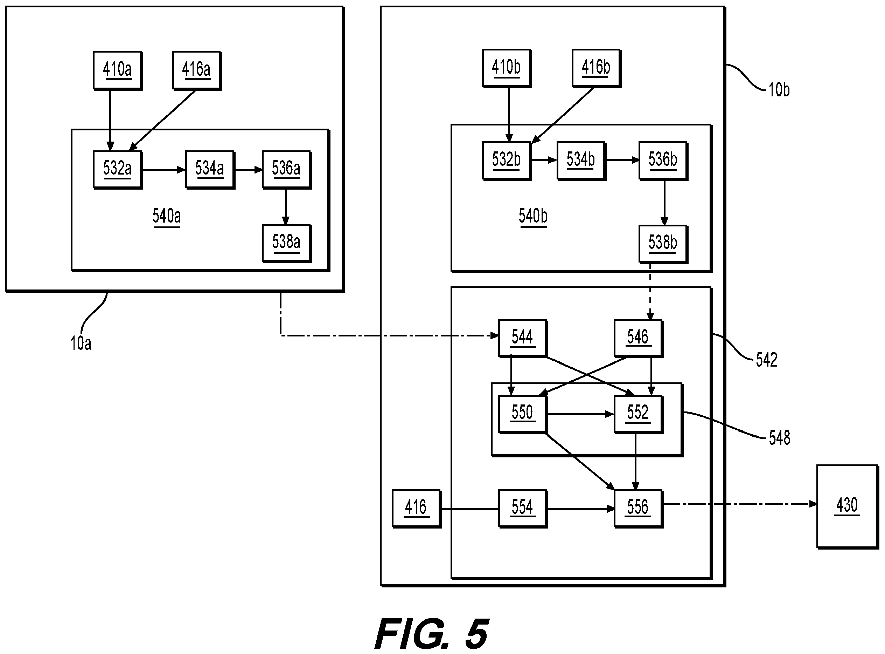

The sensors 10 have additional external connections. The power receiver/voltage regulators 402 can be connected to a charging unit 426. For example, they may be set into respective docking ports 428 for charging when required (dotted lines indicate these connections). The two BLE modules 422 can communicate wirelessly. The BLE module 410a of the sensor unit 10a may transmit data to the BLE module 422b of the sensor unit 10b, in implementations in which the sensor unit 10b acts as a master unit. This functionality will be described in more detail below with reference to Fig. 5. The BLE module 422b of the sensor unit 10b may transmit data to a mobile phone 430 or other device on which information pertaining to monitoring of the knee or other joint can be displayed, for example by interacting with an app running on the mobile phone 430. Such an app may be available for download and installation and may be specifically for interacting with the sensors 10 and to enable viewing of information relating to the joint being monitored. These transmissions are indicated by chained lines.

In operation, the accelerometers 418a,b and the gyroscopes 420a,b of the IMU 416 can take measurements relating to orientation and changes in orientation of their respective sensors 10 and movements that their respective sensors 10 undergo as a result of movement of the body parts to which they are attached. These movements reflect the use of the knee joint by the person during a time period in which the sensors 10 are attached. Together if desired with other sensed information such as readings provided by the temperature sensor(s) 410a, b, information calculated from these measurements can be provided to indicate the function of the knee over the time period. Other desired information can be indicated in conjunction with the knee function information e.g. knee temperature. In particular, it is possible to calculate the knee angle from accelerometer and gyroscope measurements, for example by calculating pitch and roll angles undergone by the sensors 10, and thus a picture of variation of the knee angle over a time period such as a day can be determined. One way to calculate knee angle using measurements from an accelerometer and a gyroscope is discussed in a paper from the 2011 IEEE International Conference on Rehabilitation Robotics entitled "Estimation of IMU and MARG orientation using a gradient descent algorithm" by Madgwick et al., the contents of which are herein incorporated by reference. Those skilled in the art will appreciate that other methods and algorithms could be used for this purpose. Moreover, the skilled reader will appreciate that other types of measurement equipment that can determine roll and pitch between two points could be used in place of the IMU 416.

Turning to Fig. 5, some functions of the sensor units 10 which facilitate the above-mentioned recording of data and performing of calculations will now be explained. Fig. 5 shows schematically some of the functions of the first and second sensors 10a, 10b. It will be understood that the functions shown and described in the following are not an exhaustive list of all the functions that may be implemented by the sensors and that the functional blocks shown are a simplified representation of some actual functions that sensors such as the sensors 10a, 10b may perform for the purpose of monitoring a joint. In a similar manner to Fig. 4, functional blocks that are common to both the sensors 10a, 10b are indicated by like reference numerals together with a sensor denotation a or b.

Both sensors 10a, 10b share some common functionality. The temperature sensor(s) 410 and the IMUs 416 are indicated by way of some measurement functions that the sensors 10a, 10b may perform. The raw measurements made by the temperature sensor(s) 410 and the IMUs 416 are provided to a data conversion unit 532, which processes the raw measurements. The processed measurements may be passed to a calibration unit 534, which can hold information relating to a calibration of the sensor units 10 which could have been performed previously, including corrections for any misalignment with the femur and tibia. Such a calibration may be updated periodically. The processed and calibrated data can be passed to an orientation estimation unit 536, which determines the orientations of the sensors 10. In some implementations, this includes the pitch and roll undergone by the sensors 10. The orientation estimation unit 536 may make use of methods such as the ones discussed above with reference to Fig. 4. The calculated parameters such as pitch and roll data can be passed to a transmitter such as an orientation data packing unit 538 for onward transmission. Such onward transmission could be by wired connections or unwired connections such as Bluetooth transmission or radio transmission etc.. The data conversion unit 532, the calibration unit 534, the orientation estimation unit 536 and the orientation data packing unit 538 can be considered to provide a functionality termed generally a sensing algorithm unit 540.

It can be seen in Fig. 5 that the second sensor 10b, which was shown in Fig. 3 mounted on the calf 13 of a patient, has some further functionality not present in the first sensor 10a, which was shown in Fig. 3 mounted to the thigh 12 of a patient. In this implementation, the second sensor 10b is a master sensor and is configured for mounting in a lower position on the patient than the first sensor 10a. It will be appreciated that the master sensor could instead be the first sensor 10a applied to the thigh of a patient and hence be configured to be mounted in an upper position to the second sensor 10b. The master sensor may be termed the fusion node and the other sensor may be termed the source node.

The further functionality present in the second sensor 10b can generally be termed a metrics algorithm unit 542. Within the metrics algorithm unit 542 are various functionalities discussed in the following. There is a receiver such as a first orientation data unpacking unit 544 arranged to receive data from the orientation data packing unit 538a of the first sensor 10a; a receiver such as a second orientation data unpacking unit 546 arranged to receive data from the orientation data packing unit 538b of the second sensor 10b. There is a calculation unit 548 which includes a knee angle estimation unit 550 and a worn classification unit 552. Both the knee angle estimation unit 550 and the worn classification unit 552 can receive data relating to angle of the sensors 10 from the first and second orientation data unpacking units 544, 546. The worn classification unit 552 can additionally receive an output from the knee angle estimation unit 550. The IMU 416 is functionally indicated again as providing an input to the metrics algorithm unit 542, specifically into a step count unit 554. The knee angle estimation unit 550, the worn classification unit 552 and the step count unit 554 are all functionally connected to an Activities of Daily Living (ADL) unit 556, which can transmit data to the mobile telephone 430 or other display device. This transmission is indicated by a chained line.

In operation, once mounted in position on a person's leg 11, the sensors can start taking measurements. One option for triggering this process is for the switches 414 to be used to switch on the sensors 10. Another additional or alternative option is for one or both of the sensors 10 to start interacting with the above-mentioned app on the phone 430. The temperature sensor(s) 10 can commence taking temperature readings. The accelerometers 418 and the gyroscopes 410 of the IMUs 416 can commence taking orientation information of the sensors 10, which may include information relating to changes in roll, pitch and, if desired, yaw angle. It is convenient for these measurements to be taken periodically so that multiple measurements can be taken over a time period in which the sensors 10 are being worn. For example, measurements may be taken 50 times/s i.e. at 50 Hz, or at some other suitable interval. The measured data passes through the various functional units common to the two sensors 10 until eventually the orientation data packing units 538 have processed it into a transmissible form. Following delivery to the metrics algorithm unit 542 within the second sensor 10b, specifically into the orientation data unpacking units 544, 546, the various functional blocks of the metrics algorithm unit 548 can process the data along with temperature data. The knee angle estimation unit 550 can use this data to calculate the knee angle at at least some of the times at which measurements were taken. The worn classification unit 552 can calculate the roll and pitch angles of the sensors 10 at at least some of the times at which measurements are taken. These calculations are then correlated in the ADL unit 556. This unit stores a summary of the metrics calculated from the obtained measurements, which may include any of the following: knee angle, step count, active time, load bearing time, and time worn. The knee angle could be stored as a value for each time measurements are taken but in practice, in order to provide a more useful output, this data may be correlated over the time period. For example, it may be stored as a histogram in 5 degree buckets in order to show the time spent in each bucket. Other possibilities for presenting the knee angle data will occur to the skilled reader. Depending on the memory capacity of the ADL 556, it may be preferable to store only the correlated data and not the raw measurements. The ADL unit 556 can operate to store data during the entirety of the time period in which the sensors 10 are worn, thereby giving an indication of the function of the knee during the patient's daily activities. This data can be uploaded to the mobile phone 130 when desired e.g.

upon request or at the end of the time period. The data could also be further correlated over multiple time periods e.g. over the course of a week. If desired, the data may also be correlated over a specific time period within the time period in which the sensors are worn, for example during a period of exercise. A shorter burst of data such as this could be streamed to the mobile phone 130. Any data uploaded to the mobile phone 430 could be processed as necessary and viewed via the app.

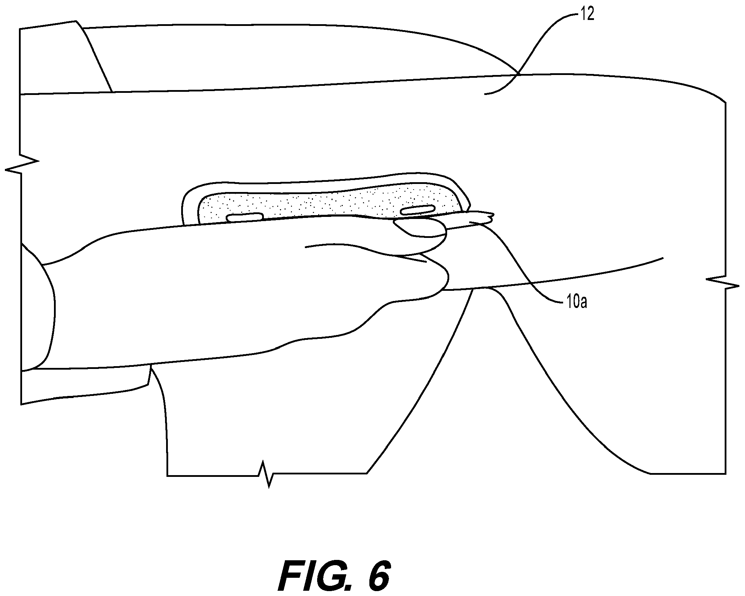

As noted above, it is undesirable to present data from measurements that are taken when one or both of the sensors 10 is not mounted in its intended position, because such data could be misleading as to the functionality of the knee. In order to address this issue, the present system includes a not worn detection system. With reference to Fig. 6, it will be appreciated that if one of the sensors 10 is removed, its orientation changes dramatically. This fact is used in the present system to enable detection of such removal. The systems and methods described in the following may be used after a previous determination that the sensors are worn e.g. after operation for any length of time as described above. Such systems and methods could also be used to determine that one or both of the sensors 10 has not yet been mounted but subsequently that they are both mounted on the leg 11 and could thus be used instead of provision of the on/off switches 414 in some circumstances. Alternatively, such systems and methods could be used as an alternative to waiting for interaction with the app on the mobile phone 430. As another alternative, such systems and methods could be used if the on/off switches 414 are used to switch on the sensors before they have been correctly mounted on the patient, so as not to take account of any measurements made before mounting is complete. They could also be used if one or both of the sensors 10 becomes dislodged from its intended position or falls off completely or to some extent. They may be used after an initial calibration of the sensors. They may also be used after an initial set up of the sensors. They could also be used after determining whether the sensors are correctly placed or that there is an error in their placement which can be corrected for. For example, as noted above, the first and second sensors 10 may include different functionality and each may be configured to be placed on a particular side of a joint in a particular orientation. Thus such systems and methods could be used to determine whether the sensors have been mounted on their correct respective body parts. Any of the possibilities discussed here could be used in any combination to determine that one or both of the sensors is not mounted and hence that the system is in an unworn state. Such systems and methods will now be described.

Referring back to Fig. 6, the exemplary first sensor 10a is being removed from the thigh 12 by peeling such that the sensor 10a undergoes a tilt. The exemplary tilt shown is a roll movement. It will be appreciated that such a movement will be at odds with the recorded movement of the other, second sensor 10b mounted on the calf 13, if the second sensor 10b is still mounted on the calf 13. This mismatch in relative orientation of the two sensors 10 can be used to determine that the sensor system 400 is in an unworn state. Even if the second sensor 10b is subsequently removed, the relative orientations of the two sensors will be different from their relative orientation when mounted across the patient's knee. This will also be the case if the second sensor is removed prior to removal of the first sensor. This will also be the case if either of the sensors is removed in a different manner e.g. by peeling with a different tilting motion such as yaw motion or by some combination of roll and pitch motion. Furthermore, the knee angle as calculated using the pitch and roll of the sensors 10 will not return a feasible knee angle in any of these situations. In other words, the observed pitch and/or roll angles and/or knee angle can be used to indicate a relative position of the body parts to which the sensors 10 are configured to be mounted i.e. in this case the thigh and calf, which is impossible or "inhuman".

An exemplary algorithm to determine an unworn state will now be described. At each time step at which measurements are taken, the worn classification unit 552 receives the current knee angle and roll angle of both sensors. Using these angles a "penalty" is calculated which is a measure of how inhuman the angles are. This penalty is calculated as follows:

aKnee, aRoll_F, aRoll_S in degrees where

k = aKnee (knee angle)

aRoll_F = roll angle of the second sensor 10b (where F indicates fusion sensor)

aRoll_S = roll angle of the first sensor 10a (where S indicates source sensor)

$$r = |aRoll\_F - aRoll\_S|$$

p_k = knee angle penalty

p_r = roll angle penalty

p = penalty

The following conditional determinations are made:

$$p\_k = 0 \text{ if } -10 <= k <= 150$$

$$= 0.5 * ((k + 10) / 160)^4 \text{ if } k < -10$$

$$= 0.5 * ((k - 150) / 160)^4 \text{ if } k > 150$$

A feasible human knee angle is anywhere between approximately 10 degrees and approximately 150 degrees so if the angle is within this range, the penalty is zero, but if it is outside this range there is a penalty.

$$p\_r = 0 \text{ if } r <= 40$$

$$= 0.5 * ((r - 40) / 140)^4 \text{ if } r > 40$$

Thus up to 40 degrees of relative roll between the thigh 12 and the calf 13 is considered feasible and the penalty is zero, but above this amount is considered unfeasible or inhuman and there is a penalty.

It should be noted that the parameters chosen for the above calculations can affect the sensitivity of the system to inhuman orientations. The present applicant has found that the more sensitive the system is set, the greater the risk of misclassification of worn/unworn state. The above parameters are shown by way of example only as ones that the applicant has determined to be advantageous following significant experimentation. Generally speaking, the parameters may be chosen such that false readings do not occur but unworn states are not missed.

$$p = p\_k + p\_r$$

Thus a combination of the roll penalty and the knee angle penalty is used to determine whether the sensor system 400 is in an unworn state.

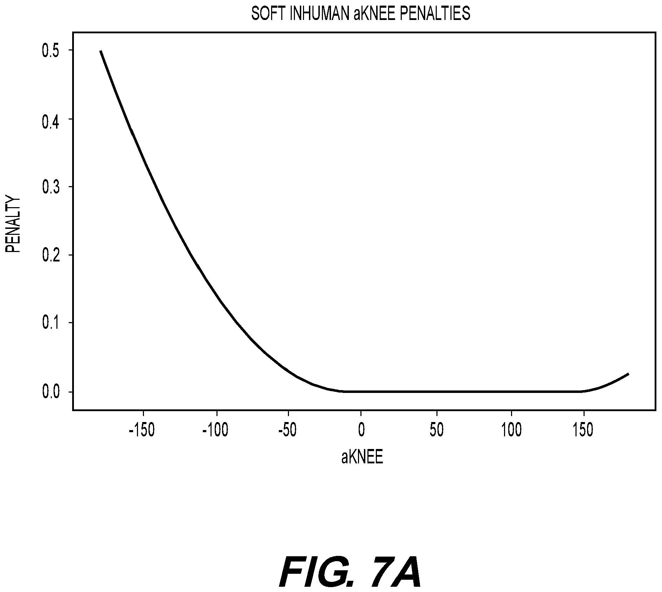

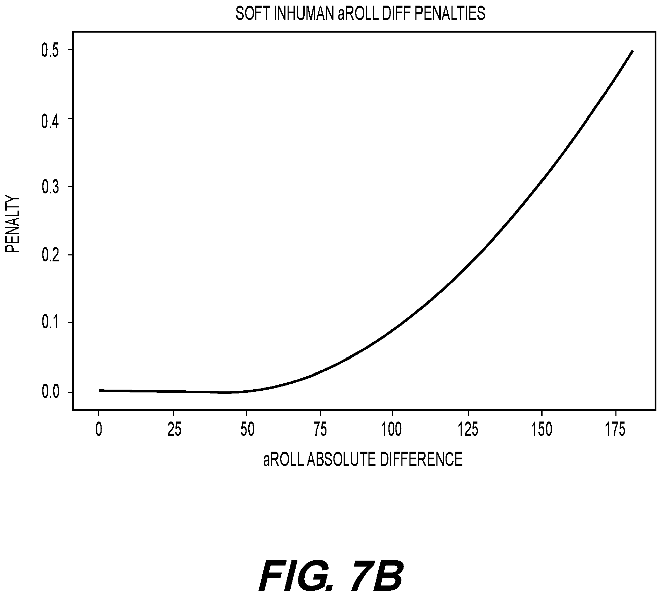

Figs. 7a & 7b show a graph of knee angle and roll angle penalties respectively as a function of the input angles. In this exemplary implementation, the final penalty is calculated as a sum of these two penalties, but a different mathematical combination of the knee and roll angle penalties could be used. The final penalty is then processed into a moving average (e.g. as an exponential recursive filter with forget rate of 0.05), which smooths the value of the final penalty over time. If this moving average goes over a threshold of 0.005, this indicates an unworn state.

Upon detection of an unworn state as described above, the sensor units, for example the metrics algorithm unit 548, can switch to a not worn state in terms of how they function. As discussed above, this is because in this situation, any data collected by the IMUs 416 will be "rogue" data, which is not indicative of the patient moving their knee. Thus in particular, it is desirable for a different procedure than the normal operation described above with respect to Figs. 4 & 5 to be adopted as regards calculation of knee angle at times subsequent to detection of the unworn state, because the picture built up over time of the patient's knee angle is an important indicator of knee health and so the system can operate to avoid skewing this data with rogue data calculated using measurements from an unworn sensor or sensors. There are various options for enabling omission of data subsequent to detection of an unworn state from being included in the information provided by the ADL unit 556 and in particular the correlated knee angle information. One option is to stop the accelerometers 418 and the gyroscopes 420 from collecting data. Another option is for the data not to be passed to the data conversion units 532, but to instead be discarded. Alternatively the data could not be transmitted from the orientation data packing units 538 or it could be transmitted but discarded upon receipt by the orientation data unpacking units 544, 546. Other options include that the knee angle is not calculated by the knee angle estimation unit 550 or that the knee angles are calculated at subsequent times but either the worn classification unit 552 does not pass on the data or the data is passed on but not included by the ADL unit 556 in its correlation process. The worn classification unit 552 could indicate the not worn state to the ADL 556 in various ways that will occur to the skilled reader.

One advantage of the present system and method is that the unworn state is detected without human intervention. Therefore, it is not reliant on a person having to remember to take any action to put the sensors into the unworn state.

After an unworn state of a sensor has been detected, alternative calculations may be made as part of the different procedure. For example, the ADL 556 could log a time duration for which the sensor system 400 is in the unworn state. This could be determined from the absence of data received or, if the data continues to be received, until the worn classification unit 552 indicates that the sensor(s) is no longer in an unworn state. In some implementations, setting the sensors 10 to charge on the charger 426 can trigger an unworn state and thus similar principles could be used to measure duration of time for which the sensors are charged. If the calculated penalty falls back to below the threshold, the sensor system 400 can return to normal operation. In some implementations, the sensor system 400 is set to await a user input before recommencing normal operation e.g. operation of the on/off switches on the sensors 10 or the master sensor 10b interacting with the app on the mobile telephone 430. In this way, the system 400 will only recommence normal operation when the sensors 10 have been placed in position across the joint. In other implementations, an automatic return to normal operation could be implemented when the calculated penalty falls back to below the threshold, but it will be appreciated that it is possible for the two sensors 10a, 10b to be disposed relative to each other as they would be when fitted across a joint, even when not actually fitted to a joint. Thus in order to prevent normal operation recommencing in this situation, some further information would need to be gathered e.g. detection of skin contact.

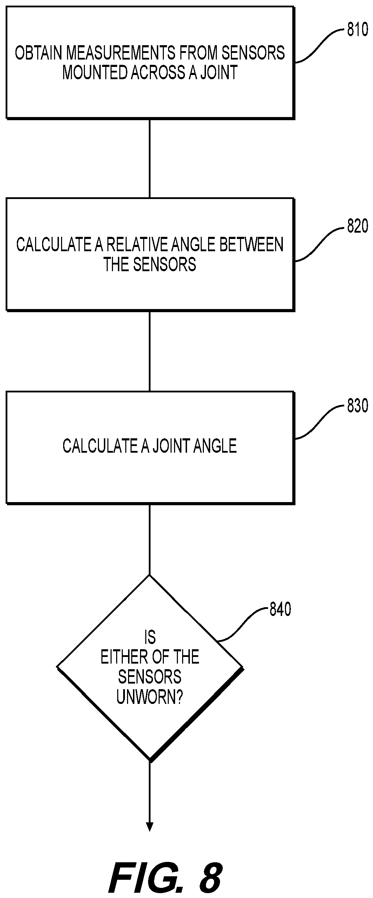

Fig. 8 shows a flowchart of a method in accordance with implementations of the invention. At 810, measurements are obtained from a pair of sensors mounted across a joint. For example, these could be the sensors 10 mounted on a thigh and calf across a knee joint of a patient.

At 812, the obtained measurements are used to calculate a relative angle between the sensors. In examples discussed above, this is performed by the worn classification functional unit 552. This could be an absolute difference in angle about any of the roll, pitch or yaw axes. In examples described above, the angle is roll. This calculation is simplified by the sensors 10 having both been placed on a same side of the leg 11 such that both lie substantially in the plane of the knee angle.

At 814, the obtained measurements are used to calculate a joint angle. In examples discussed above, this is performed by the knee angle estimation functional unit 550. In examples described above, the measurements may have been used to calculate roll and pitch angles and these values can be used to calculate the knee angle. Thus the obtained measurements are used indirectly to calculate the knee angle.

At 816, the relative angle and knee angle calculated are used to determine whether either of the sensors is unworn. In the examples discussed above, this determination could be made in the worn classification functional unit 552, which has determined roll angle at 812 and which can receive knee angle from the knee angle estimation functional unit 550. It could alternatively be made in the ADL unit 556 or by a separate functional unit.

As discussed above, if an unworn state is detected, various actions can be taken by the system to vary the procedure adopted subsequently with regard to data provided about the knee. As also discussed above, a subsequent return to normal operation could be implemented.

It will be appreciated that many variations to the above-discussed examples could be made without departing from the principles set out above.

For example, the functional units could be arranged differently and the calculations performed in a different order or by means of variations in the exemplary methods described above. The sensor components described above with reference to Fig. 4 could be different types or be used in different combinations and some of the described components may be provided as a single component. Whilst many examples above use relative roll angle between the two sensors 10, this is not essential and relative pitch or relative yaw could instead be used. Also, as mentioned above, in some implementations, mounting of the sensors on the wrong limbs can trigger an unworn state of the system, because doing this would result in a knee angle indicating that the knee is bending the wrong way. However, as an alternative to triggering an unworn state in this scenario, a different state could be triggered, the actual mounting position determined and taken into account during processing of the data in otherwise normal operation. In some implementations, the unworn detection system could be disabled prior to normal operation of the sensors, and a different procedure used to determine that the sensors are on the wrong limbs, to thereby trigger the different state. The unworn detection system could then be enabled so that subsequent removal etc. of the sensors would be detected.

In other variations, more than two sensors could be used. For example, it may be desirable to use three sensors if the joint under surveillance is a ball and socket joint which has three degrees of freedom of movement. In some cases, it may be desirable to use an additional sensor on a joint such as a knee joint to assist in determining orientations of the thigh and calf. For example, two sensors could be placed on one of the user's limbs. Measurements from such a third sensor could be processed in a similar manner to the processing described above for two sensors. One possibility is to process the data from the two sensors placed on one limb to obtain a set of data for that limb, and then process that in conjunction with the data from the other limb as described above.

The functions of the microcontroller 424 described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor such as the microcontroller 424, which can be special or general purpose, coupled to receive data and instructions from, and to transmit data and instructions to storage devices such as sticks. Such computer programs, which can be software, software applications, applications, components, or code, include machine instructions for a programmable processor such as the microcontroller 424, and can be implemented in a high-level procedural and/or object-oriented programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor and which can receive instructions as a machine-readable signal. Such a machine-readable medium can store instructions transitorily or non-transitorily.

## Claims

1. A method for determining an unworn state of a sensor system (400) comprising a pair of sensors (10a, 10b) configured for mounting on first (12) and second (13) body parts either side of a joint (4), wherein each sensor (10a; 10b) contains one or more motion sensing devices which permit either {i} the pitch and/or roll of the individual sensor (10a; 10b) to be determined or {ii} the relative pitch and/or roll between the sensors (10a, 10b) to be determined, the method comprising:

obtaining (810) one or more measurements from each sensor (10a; 10b);
calculating (820) a relative angle between the two sensors (10a, 10b) using one or more of the obtained one or more measurements;
calculating (830), using one or more of the obtained one or more measurements, a joint angle between the first and second body parts, where the joint angle is defined in a plane of normal bending of the joint; and
determining (840), based on the calculated angles, whether either or both of the sensors (10a; 10b) is not mounted and if so, determining that the system is in an unworn state.

2. The method of claim 1, wherein obtaining (810) one or more measurements from each sensor (10a, 10b) is performed multiple times in a time period and the calculating (820) a relative angle and the calculating (830) a joint angle is performed using measurements obtained at at least some of the multiple times, the method comprising the further step of, following a determination (840) that the sensor system is in an unworn state, adopting a different procedure with regard to calculating the joint angle at subsequent times, wherein, optionally, adopting a different procedure comprises any one or more of: not calculating the joint angle at subsequent times; differently processing the calculated joint angles at subsequent times from those calculated at times prior to the determination; not storing the joint angles calculated at subsequent times.

3. The method of claim 2, further comprising correlating the joint angles and relative angles calculated at multiple times to provide information about the joint (4) over the time period, and wherein adopting a different procedure comprises omitting any data calculated using measurements made at subsequent times from the information, wherein, optionally, the information comprises any one or more of: joint angle variation during the time period; duration of time in which the joint (4) is active; duration of time in which the joint is bearing a load; length of the time period; and if the joint (4) is a knee, step count over the time period.

4. The method of claim 3, wherein adopting a different procedure comprises performing calculations to provide different information from the information about the joint (4), wherein the different information includes any one or more of: unworn time; and if the first sensor (10a) is determined to be charging, charging time.

5. The method of any preceding claim, wherein an unworn state includes any one or more of: the first sensor having been at least partially removed from its mounted position; the first sensor falling off its mounted position at least to some extent; the first sensor being switched on but not mounted on the first body part; the second sensor being switched on but not mounted on the second body part.

6. The method of any preceding claim, wherein the determining whether either or both of the sensors is not mounted includes determining if the first sensor (10a) is mounted on the first body part (12) which comprises calculating a value of a function of the calculated angles.

7. The method of claim 6, further comprising processing multiple calculated values of the function over a time period to determine a moving average value for the function and wherein the determining further comprises comparing the determined moving average value to a threshold value.

8. The method of claim 6 or claim 7, wherein the function comprises a function of a joint angle penalty and a relative angle penalty, wherein, optionally,

(i) if the joint angle is within a feasible range for the joint (4), the joint angle penalty is zero and if the joint angle is not within a feasible range for the joint (4), the joint angle penalty has a value which depends on how far outside the feasible range the joint angle is; and/or
(ii) if the relative angle is less than or equal to a threshold relative value, the relative angle penalty is zero and if the relative angle penalty is greater than the threshold relative value, the relative angle penalty depends on how much greater the relative angle is than the threshold relative value.

9. The method of any preceding claim, wherein the relative angle is a difference in tilt angle of the two body parts, where tilting occurs about an axis perpendicular or substantially perpendicular to the plane in which the joint angle is defined, wherein, optionally, the joint angle is defined in an x-z plane and the tilt angle is defined in an x-y plane and is non-zero if the first and second body parts undergo a relative roll about the x-axis.

10. The method of any preceding claim, further comprising, prior to the obtaining (810),

(i) determining whether the first and second sensors are mounted on correct respective first and second body parts (12; 14) and/or in substantially a predetermined orientation relative to the correct respective first and second body parts (12; 14), and if one or both sensors (10a; 10b) is determined to be mounted on the wrong body part or in a different orientation from the predetermined orientation, adjusting the calculating steps to take account of an actual determined mounting location and/or orientation; and/or
(ii) calibrating the first and second sensors with respect to a predetermined orientation relative to the respective first and second body parts (12; 14).

11. The method of any preceding claim, wherein the joint (4) is a knee or an elbow.

12. A system (400) for providing information about a joint (4), comprising:

 a first sensor unit (10a) configured to be mounted on a first body part (12) on a first side of a joint (4), the first sensor unit (10a) comprising:

  one or more first sensors (10a) arranged to take one or more measurements relating to the first body part (12) and one or more measurements relating to the first sensor (10a); and
  a transmitter arranged to transmit taken measurements; and

 a second, master sensor unit (10b) configured to be mounted on a second body part (12) on a second side of a joint (4), the second master sensor unit (10b) comprising:

  one or more second sensors (10b) arranged to take one or more measurements relating to the second body part (14) and one or more measurements relating to the second sensor (10b);
  a receiver arranged to receive measurements transmitted from the first sensor unit transmitter; and
  a calculation unit configured to calculate, using one or more of the measurements, a relative angle between the two sensors (10a, 10b) and a joint angle between the first and second body parts (12, 14), where the joint angle is defined in a plane of normal bending of the joint (4), the calculation unit being further configured to determine, based on the calculated angles, whether either or both of the sensor units (10a, 10b) is not mounted and if so, determine that the system (400) is in an unworn state, wherein each of the one or more first sensors (10a; 10b) contains one or more motion sensing devices which permit either {i} the pitch and/or roll of the individual first sensor (10a; 10b) to be determined or {ii} the relative pitch and/or roll between the first sensor and a second sensor (10a, 10b) to be determined, and wherein each of the one or more second sensors (10a; 10b) contains one or more motion sensing devices which permit either {i} the pitch and/or roll of the individual second sensor (10a; 10b) to be determined or {ii} the relative pitch and/or roll between the second sensor and a first sensor (10a, 10b) to be determined.

13. The system of claim 12, wherein the first and second sensor units (10a, 10b) are configured to take measurements multiple times in a time period and the calculation unit is configured to calculate a relative angle and a joint angle using measurements obtained at each of at least some of the multiple times, the calculation unit being further configured to, following a determination that the system (400) is in an unworn state, adopt a different procedure with regard to calculating the joint angle at subsequent times.

14. The system of claim 13, wherein the calculation unit is further configured to correlate the joint angles and relative angles calculated at multiple times to provide information about the joint (4) over the time period, and wherein adopting a different procedure comprises omitting any data relating to measurements made at subsequent times from the information, wherein, optionally,

 (i) the second, master sensor unit is configured to provide the information to a mobile device for viewing by a user; and/or
 (ii) the information is for use in assessing health and/or rehabilitation of the joint (4).

15. The system of any of claims 12 to 14, wherein the first and second sensor units (10a, 10b) are generally planar having front and back faces and are configured for their back faces to be mounted on the first and second body parts (12, 14) at a side of the joint substantially parallel to the plane of normal bending of the joint (4).

**Patentansprüche**

1. Verfahren zum Bestimmen eines ungetragenen Zustands eines Sensorsystems (400), das ein Paar von Sensoren (10a, 10b) umfasst, die konfiguriert sind, um an einem ersten (12) und einem zweiten (13) Körperteil auf beiden Seiten eines Gelenks (4) angebracht zu werden, wobei jeder Sensor (10a; 10b) eine oder mehrere bewegungserfassende Vorrichtungen enthält, die es ermöglichen, entweder {i} das Nicken und/oder das Rollen des einzelnen Sensors (10a; 10b) zu bestimmen oder {ii} das relative Nicken und/oder Rollen zwischen den Sensoren (10a, 10b) zu bestimmen, wobei das Verfahren umfasst:

Erlangen (810) einer oder mehrerer Messungen von jedem Sensor (10a; 10b);
Berechnen (820) eines relativen Winkels zwischen den beiden Sensoren (10a, 10b) unter Verwendung einer oder mehrerer der erlangten einen oder mehreren Messungen;
Berechnen (830), unter Verwendung einer oder mehrerer der erlangten einen oder mehreren Messungen, eines Gelenkwinkels zwischen dem ersten und dem zweiten Körperteil, wobei der Gelenkwinkel in einer Ebene normaler Beugung des Gelenks definiert ist; und
Bestimmen (840), basierend auf den berechneten Winkeln, ob einer oder beide Sensoren (10a; 10b) nicht angebracht sind, und wenn das der Fall ist, Bestimmen, dass sich das System in einem ungetragenen Zustand befindet.

2. Verfahren nach Anspruch 1, wobei das Erlangen (810) einer oder mehrerer Messungen von jedem Sensor (10a, 10b) mehrere Male in einem Zeitraum durchgeführt wird und das Berechnen (820) eines relativen Winkels und das Berechnen (830) eines Gelenkwinkels unter Verwendung von Messungen durchgeführt werden, die zumindest bei einigen der mehreren Male erhalten wurden, wobei das Verfahren nach einem Bestimmen (840), dass sich das Sensorsystem in einem ungetragenen Zustand befindet, den weiteren Schritt umfasst: Anwenden eines anderen Verfahrens in Bezug auf das Berechnen des Gelenkwinkels zu nachfolgenden Zeitpunkten, wobei optional das Anwenden eines anderen Verfahrens eines oder mehrere der folgenden umfasst: kein Berechnen des Gelenkwinkels zu nachfolgenden Zeitpunkten; unterschiedliches Verarbeiten der berechneten Gelenkwinkel zu nachfolgenden Zeitpunkten im Vergleich zu jenen, die zu Zeitpunkten vor der Bestimmung berechnet wurden; kein Speichern der zu nachfolgenden Zeitpunkten berechneten Gelenkwinkel.

3. Verfahren nach Anspruch 2, das ferner ein Korrelieren der zu mehreren Zeitpunkten berechneten Gelenkwinkel und relativen Winkel umfasst, um Informationen über das Gelenk (4) über den Zeitraum bereitzustellen, und wobei das Anwenden eines anderen Verfahrens das Weglassen jeglicher Daten umfasst, die unter Verwendung von zu späteren Zeitpunkten durchgeführten Messungen aus den Informationen berechnet wurden, wobei, optional, die Informationen eines oder mehrere der folgenden umfassen: Variation des Gelenkwinkels während des Zeitraums; Dauer der Zeit, in der das Gelenk (4) aktiv ist; Dauer der Zeit, in der das Gelenk eine Last trägt; Länge des Zeitraums; und, wenn das Gelenk (4) ein Knie ist, Schrittzahl über den Zeitraum.

4. Verfahren nach Anspruch 3, wobei das Anwenden eines anderen Verfahrens ein Durchführen von Berechnungen umfasst, um aus den Informationen über das Gelenk (4) andere Informationen bereitzustellen, wobei die anderen Informationen eine oder mehrere der folgenden umfassen: die Zeit des Nichttragens; und, wenn der erste Sensor (10a) als aufladend bestimmt wird, die Aufladezeit.

5. Verfahren nach einem der vorhergehenden Ansprüche, wobei ein ungetragener Zustand eines oder mehrere der folgenden aufweist: der erste Sensor wurde zumindest teilweise aus seiner angebrachten Position entfernt; der erste Sensor fällt zumindest teilweise aus seiner angebrachten Position; der erste Sensor ist eingeschaltet, aber nicht am ersten Körperteil angebracht; der zweite Sensor ist eingeschaltet, aber nicht am zweiten Körperteil angebracht.

6. Verfahren nach einem der vorhergehenden Ansprüche, wobei das Bestimmen, ob einer oder beide Sensoren nicht angebracht sind, ein Bestimmen umfasst, ob der erste Sensor (10a) an dem ersten Körperteil (12) angebracht ist, was ein Berechnen eines Wertes einer Funktion der berechneten Winkel umfasst.

7. Verfahren nach Anspruch 6, das ferner ein Verarbeiten mehrerer berechneter Werte der Funktion über einen Zeitraum umfasst, um einen gleitenden Mittelwert für die Funktion zu bestimmen, wobei das Bestimmen ferner ein Vergleichen des bestimmten gleitenden Mittelwerts mit einem Schwellenwert umfasst.

8. Verfahren nach Anspruch 6 oder Anspruch 7, wobei die Funktion eine Funktion einer gemeinsamen Winkelstrafe und einer relativen Winkelstrafe umfasst, wobei optional:

(i) wenn der Gelenkwinkel innerhalb eines durchführbaren Bereichs für das Gelenk (4) liegt, die Gelenkwinkelstrafe Null beträgt, und wenn der Gelenkwinkel nicht innerhalb eines durchführbaren Bereichs für das Gelenk (4) liegt, die Gelenkwinkelstrafe einen Wert aufweist, der davon abhängt, wie weit außerhalb des durchführbaren Bereichs der Gelenkwinkel liegt; und/oder
(ii) wenn der relative Winkel kleiner oder gleich einem relativen Schwellenwert ist, die relative Winkelstrafe Null beträgt, und wenn die relative Winkelstrafe größer als der relative Schwellenwert ist, die relative Winkelstrafe davon abhängt, wie viel größer der relative Winkel als der relative Schwellenwert ist.

9. Verfahren nach einem der vorhergehenden Ansprüche, wobei der relative Winkel eine Differenz im Neigungswinkel der beiden Körperteile ist, wobei das Neigen um eine Achse erfolgt, die senkrecht oder im Wesentlichen senkrecht auf der Ebene steht, in der der Gelenkwinkel definiert ist, wobei, optional, der Gelenkwinkel in einer x-z-Ebene definiert ist und der Neigungswinkel in einer x-y-Ebene definiert ist und nicht Null beträgt, wenn der erste und der zweite Körperteil ein relatives Rollen um die x-Achse erfahren.

10. Verfahren nach einem der vorhergehenden Ansprüche, das ferner vor dem Erlangen (810) umfasst:

    (i) Bestimmen, ob der erste und der zweite Sensor an korrekten jeweiligen ersten und zweiten Körperteilen (12; 14) und/oder in im Wesentlichen einer vorbestimmten Ausrichtung relativ zu den korrekten jeweiligen ersten und zweiten Körperteilen (12; 14) angebracht sind, und wenn festgestellt wird, dass einer oder beide Sensoren (10a; 10b) an dem falschen Körperteil oder in einer anderen Ausrichtung als der vorbestimmten Ausrichtung angebracht sind, Anpassen der Berechnungsschritte, um eine tatsächliche bestimmte Anbringungsstelle und/oder Ausrichtung zu berücksichtigen; und/oder
    (ii) Kalibrieren des ersten und des zweiten Sensors in Bezug auf eine vorbestimmte Ausrichtung relativ zu dem jeweiligen ersten und zweiten Körperteil (12; 14).

11. Verfahren nach einem der vorhergehenden Ansprüche, wobei das Gelenk (4) ein Knie oder ein Ellbogen ist.

12. System (400) zur Bereitstellung von Informationen über ein Gelenk (4), das umfasst:

    eine erste Sensoreinheit (10a), die konfiguriert ist, um an einem ersten Körperteil (12) auf einer ersten Seite eines Gelenks (4) angebracht zu werden, wobei die erste Sensoreinheit (10a) umfasst:

        einen oder mehrere erste Sensoren (10a), der oder die eingerichtet ist oder sind, um eine oder mehrere Messungen in Bezug auf den ersten Körperteil (12) und eine oder mehrere Messungen in Bezug auf den ersten Sensor (10a) vorzunehmen; und
        einen Sender, der eingerichtet ist, um die vorgenommenen Messungen zu übertragen; und

    eine zweite, übergeordnete Sensoreinheit (10b), die konfiguriert ist, um an einem zweiten Körperteil (12) auf einer zweiten Seite eines Gelenks (4) angebracht zu werden, wobei die zweite übergeordnete Sensoreinheit (10b) umfasst:

        einen oder mehrere zweite Sensoren (10b), der oder die eingerichtet ist oder sind, um eine oder mehrere Messungen in Bezug auf den zweiten Körperteil (14) und eine oder mehrere Messungen in Bezug auf den zweiten Sensor (10b) vorzunehmen;
        einen Empfänger, der eingerichtet ist, um die von dem Sender der ersten Sensoreinheit gesendeten Messwerte zu empfangen; und
        eine Berechnungseinheit, die konfiguriert ist, um unter Verwendung einer oder mehrerer der Messungen einen relativen Winkel zwischen den beiden Sensoren (10a, 10b) und einen Gelenkwinkel zwischen dem ersten und dem zweiten Körperteil (12, 14) zu berechnen, wobei der Gelenkwinkel in einer Ebene normaler Beugung des Gelenks (4) definiert ist, wobei die Berechnungseinheit ferner konfiguriert ist, um auf der Grundlage der berechneten Winkel zu bestimmen, ob einer oder beide der Sensoreinheiten (10a, 10b) nicht angebracht ist oder sind, und wenn dies der Fall ist, zu bestimmen, dass sich das System (400) in einem ungetragenen Zustand befindet,
    wobei jeder des einen oder der mehreren ersten Sensoren (10a; 10b) eine oder mehrere Bewegungserfassungsvorrichtungen enthält, die es erlaubt oder erlauben, entweder {i} das Nicken und/oder Rollen des einzelnen ersten Sensors (10a; 10b) zu bestimmen oder {ii} das relative Nicken und/oder Rollen zwischen dem ersten Sensor und einem zweiten Sensor (10a, 10b) zu bestimmen, und wobei jeder des einen oder der mehreren zweiten Sensoren (10a; 10b) eine oder mehrere bewegungserfassende Vorrichtungen enthält, die es erlaubt oder erlauben, entweder {i} das Nicken und/oder Rollen des einzelnen zweiten Sensors (10a; 10b) zu bestimmen oder {ii} das relative Nicken und/oder Rollen zwischen dem zweiten Sensor und einem ersten Sensor (10a, 10b) zu bestimmen.

13. System nach Anspruch 12, wobei die erste und die zweite Sensoreinheit (10a, 10b) konfiguriert sind, um in einem Zeitraum Messungen mehrere Male vorzunehmen, und die Berechnungseinheit konfiguriert ist, um einen relativen Winkel und einen Gelenkwinkel unter Verwendung von Messungen zu berechnen, die zu jedem von zumindest einigen der mehreren Male erhalten werden, wobei die Berechnungseinheit ferner konfiguriert ist, um nach einer Bestimmung, dass sich das System (400) in einem ungetragenen Zustand befindet, ein anderes Verfahren in Bezug auf die Berechnung des Gelenkwinkels zu nachfolgenden Zeitpunkten anzuwenden.

14. System nach Anspruch 13, wobei die Berechnungseinheit ferner konfiguriert ist, um die Gelenkwinkel und die relativen Winkel, die zu den mehreren Malen berechnet wurden, zu korrelieren, um Informationen über das Gelenk (4) über den Zeitraum bereitzustellen, und wobei das Anwenden eines anderen Verfahrens das Weglassen jeglicher Daten, die sich auf Messungen beziehen, die zu späteren Zeitpunkten vorgenommen wurden, aus den Informationen umfasst, wobei, optional:

> (i) die zweite übergeordnete Sensoreinheit konfiguriert ist, um die Informationen an eine mobile Vorrichtung zur Anzeige durch einen Benutzer bereitzustellen; und/oder
> (ii) die Informationen der Beurteilung der Gesundheit und/oder der Rehabilitation des Gelenks (4) dienen.

15. System nach einem der Ansprüche 12 bis 14, wobei die erste und die zweite Sensoreinheit (10a, 10b) im Allgemeinen planar sind und eine Vorder- und eine Rückseite aufweisen und so konfiguriert sind, dass ihre Rückseiten auf dem ersten und dem zweiten Körperteil (12, 14) an einer Seite des Gelenks im Wesentlichen parallel zur Ebene der normalen Beugung des Gelenks (4) angebracht werden.

## Revendications

1. Procédé de détermination d'un état non porté d'un système de capteurs (400) comprenant une paire de capteurs (10a, 10b) conçus pour être fixés sur des première (12) et seconde (13) parties du corps de part et d'autre d'une articulation (4), dans lequel chaque capteur (10a, 10b) contient un ou plusieurs dispositifs de détection de mouvement qui permettent soit {i} de déterminer le tangage et/ou le roulis du capteur individuel (10a, 10b) soit {ii} de déterminer le tangage et/ou le roulis relatif entre les capteurs (10a, 10b), le procédé comprenant:

> l'obtention (810) d'une ou plusieurs mesures à partir de chaque capteur (10a; 10b);
> le calcul (820) d'un angle relatif entre les deux capteurs (10a, 10b) à l'aide d'une ou plusieurs parmi la ou les mesures obtenues;
> le calcul (830), à l'aide d'une ou plusieurs parmi la ou les mesures obtenues, d'un angle d'articulation entre les première et seconde parties du corps, là où l'angle d'articulation est défini dans un plan de pliage normal de l'articulation; et la détermination (840), sur la base des angles calculés, si l'un ou l'autre ou les deux capteurs (10a; 10b) ne sont pas fixés et si tel est le cas, la détermination du fait que le système se trouve dans un état non porté.

2. Procédé selon la revendication 1, dans lequel l'obtention (810) d'une ou plusieurs mesures provenant de chaque capteur (10a; 10b) est réalisée à plusieurs moments dans une période de temps et le calcul (820) d'un angle relatif et le calcul (830) d'un angle d'articulation est réalisé à l'aide des mesures obtenues à certains moments parmi les multiples moments, le procédé comprenant l'autre étape consistant à, suite à une détermination (840) que le système de capteurs n'est pas porté, adopter une procédure différente quant au calcul de l'angle d'articulation à des moments successifs, dans lequel, éventuellement, l'adoption d'une procédure différente comprend l'une quelconque ou plusieurs actions parmi: le non calcul de l'angle d'articulation à des moments successifs, le traitement différent des angles d'articulation calculés à des moments successifs de ceux calculés à des moments avant la détermination; le non stockage des angles d'articulation calculés à des moments successifs.

3. Procédé selon la revendication 2, comprenant en outre la corrélation des angles d'articulation et des angles relatifs calculés à de multiples moments pour fournir des informations concernant l'articulation (4) sur la période de temps, et dans lequel l'adoption d'une procédure différente comprend l'omission de toutes les données calculées à l'aide des mesures faites à des moments successifs à partir des informations, dans lequel, éventuellement, les informations comprennent l'un ou plusieurs des éléments: variation d'angle d'articulation pendant la période de temps; durée pendant laquelle l'articulation (4) est active, durée pendant laquelle l'articulation supporte une charge, durée de la période de temps; et si l'articulation (4) est le genou, le comptage de pas pendant la période de temps.

4. Procédé selon la revendication 3, dans lequel l'adoption d'une procédure différente comprend la réalisation de calculs pour fournir différentes informations à partir des informations concernant l'articulation (4), dans lequel les différentes informations comprennent l'un ou plusieurs des éléments parmi: durée de l'état non porté et s'il est déterminé que le premier capteur (10a) est en charge, le temps de charge.

5. Procédé selon l'une quelconque revendication précédente, dans lequel un état non porté comprend l'un ou plusieurs éléments parmi: le premier capteur a été au moins partiellement retiré de sa position de fixation; le premier capteur tombe de sa position de fixation au moins dans une certaine mesure; le premier capteur est allumé mais n'est pas fixé à la première partie du corps, le second capteur est allumé mais n'est pas fixé sur la seconde partie du corps.

6. Procédé selon l'une quelconque revendication précédente, dans lequel la détermination si l'un ou l'autre ou les deux des capteurs n'est/ne sont pas fixé(s) consiste à déterminer si le premier capteur (10a) est fixé à la première partie de corps (12) qui comprend le calcul d'une valeur d'une fonction des angles calculés.

7. Procédé selon la revendication 6, comprenant en outre le traitement de plusieurs valeurs calculées de la fonction sur une période de temps pour déterminer une valeur moyenne de déplacement pour la fonction et dans lequel la détermination consiste en outre à comparer la valeur moyenne de déplacement déterminée avec une valeur seuil.

8. Procédé selon la revendication 6 ou la revendication 7, dans lequel la fonction comprend une fonction d'une pénalité d'angle d'articulation et une pénalité d'angle relatif, dans lequel, éventuellement,

   (i) si l'angle d'articulation se situe dans une plage réalisable pour l'articulation (4), la pénalité d'angle d'articulation est nulle et si l'angle d'articulation ne se situe pas dans une plage réalisable pour l'articulation (4), la pénalité d'angle d'articulation a une valeur qui dépend de la distance à laquelle l'angle de l'articulation se trouve en dehors de la plage réalisable ; et/ou
   (ii) si l'angle relatif est inférieur ou égal à une valeur seuil, la pénalité d'angle relatif est nulle et si la pénalité d'angle relatif est supérieure à la valeur relative seuil, la pénalité d'angle relatif dépend de l'amplitude de l'angle relatif par rapport à la valeur relative seuil.

9. Procédé selon l'une quelconque revendication précédente, dans lequel l'angle relatif est une différence d'angle d'inclinaison des deux parties du corps, là où l'inclinaison se produit autour d'un axe perpendiculaire ou sensiblement perpendiculaire au plan dans lequel est défini l'angle d'articulation, dans lequel, éventuellement, l'angle d'articulation est défini dans un plan x-z et l'angle d'inclinaison est défini dans un plan x-y et est non nul si les première et seconde parties de corps sont soumises à un roulis relatif autour d'un axe x.

10. Procédé selon l'une quelconque revendication précédente, comprenant en outre, avant l'obtention (810),

   (i) la détermination si les premier et second capteurs sont fixés sur les première et seconde parties du corps correctes respectives (12; 14) et/ou dans une orientation sensiblement prédéterminée par rapport aux première et seconde parties du corps correctes respectives (12; 14), et s'il est déterminé que l'un ou les deux capteurs (10a; 10b) sont fixés sur la partie du corps incorrecte ou dans une orientation différente de l'orientation prédéterminée, l'ajustement des étapes de calcul pour prendre en compte un emplacement de fixation déterminé et/ou une orientation; et/ou
   (ii) l'étalonnage des premier et second capteurs par rapport à une orientation prédéterminée relative aux première et seconde parties du corps respectives (12; 14).

11. Procédé selon l'une quelconque revendication précédente, dans lequel l'articulation (4) est un genou ou un coude.

12. Système (400) permettant de fournir des informations concernant une articulation (4), comprenant:

   une première unité de capteur (10a) conçue pour être fixée sur une première partie du corps (12) sur un premier côté d'une articulation (4), la première unité de capteur (10a) comprenant:

      un ou plusieurs premiers capteurs (10a) conçus pour prendre une ou plusieurs mesures relatives à la première partie du corps (12) et une ou plusieurs mesures relatives au premier capteur (10a); et
      un émetteur conçu pour transmettre les mesures prises; et

   une seconde unité de capteur maître (10b) conçue pour être fixée sur une seconde partie du corps (12) sur un second côté d'une articulation (4), la seconde unité de capteur maître (10b) comprenant:

      un ou plusieurs seconds capteurs (10b) conçus pour prendre une ou plusieurs mesures relatives à la seconde partie du corps (14) et une ou plusieurs mesures relatives au second capteur (10b);
      un récepteur conçu pour recevoir les mesures transmises par l'émetteur de la première unité de capteur; et
      une unité de calcul conçue pour calculer, à l'aide d'une ou plusieurs parmi les mesures, un angle relatif entre les deux capteurs (10a, 10b) et un angle d'articulation entre les première et seconde parties du corps (12, 14), l'angle d'articulation étant défini dans un plan de pliage normal de l'articulation (4), l'unité de calcul étant en outre conçue pour déterminer, sur la base des angles calculés, si l'une ou l'autre ou les deux parmi les unités de capteur (10a, 10b) ne sont pas fixées et si tel est le cas, déterminer que le système (400) se trouve dans un état non porté,

dans lequel chacun parmi le ou les premiers capteurs (10a, 10b) contient un ou plusieurs dispositifs de détection de mouvement qui permettent de déterminer soit {i} le tangage et/ou le roulis du premier capteur individuel (10a, 10b) soit {ii} le tangage et/ou le roulis relatif entre le premier et un second capteur (10a, 10b), et dans lequel chacun parmi le ou les seconds capteurs (10a, 10b) contient un ou plusieurs dispositifs de détection de mouvement qui permettent de déterminer soit {i} le tangage et/ou le roulis du second capteur individuel (10a, 10b) soit {ii} le tangage et/ou le roulis relatif entre le second et un premier capteur (10a, 10b).

13. Système selon la revendication 12, dans lequel les première et seconde unités de capteur (10a, 10b) sont conçues pour prendre des mesures à plusieurs moments dans une période de temps et l'unité de calcul est conçue pour calculer un angle relatif et un angle d'articulation à l'aide des mesures obtenues à chacun d'au moins certains des moments multiples, l'unité de calcul étant en outre conçue pour, suite au fait qu'il a été déterminé que le système (400) se trouve dans un état non porté, adopter une procédure différente quant au calcul de l'angle d'articulation à des moments successifs.

14. Système selon la revendication 13, dans lequel l'unité de calcul est en outre conçue pour corréler les angles d'articulation et les angles relatifs calculés à plusieurs moments pour fournir des informations concernant l'articulation (4) sur la période de temps, et dans lequel l'adoption d'une procédure différente consiste à omettre toutes données relatives aux mesures faites à des moments successifs à partir des informations, dans lequel, éventuellement,

(i) la seconde unité de capteur maître est conçue pour fournir les informations à un dispositif mobile que pourra consulter un utilisateur; et/ou
(ii) les informations sont destinées à être utilisées dans l'évaluation de la santé et/ou de la rééducation de l'articulation (4).

15. Système selon l'une quelconque des revendications 12 à 14, dans lequel les première et seconde unités de capteur (10a, 10b) sont en règle générale planes, dotées de faces avant et arrière et sont conçues de manière à ce que leurs faces arrière puissent être fixées sur les première et seconde parties du corps (12, 14) sur un côté de l'articulation sensiblement parallèle au plan du pliage normal de l'articulation (4).

**FIG. 1**

**FIG. 2**

**FIG. 3**

FIG. 4

*FIG. 5*

12

10a

*FIG. 6*

SOFT INHUMAN aKNEE PENALTIES

FIG. 7A

FIG. 7B

```
┌─────────────────────────────────────┐
│                                      │
│   OBTAIN MEASUREMENTS FROM SENSORS   │ ──810
│      MOUNTED ACROSS A JOINT          │
│                                      │
└─────────────────────────────────────┘
                   │
                   │
┌─────────────────────────────────────┐
│                                      │
│   CALCULATE A RELATIVE ANGLE BETWEEN │ ──820
│            THE SENSORS               │
│                                      │
└─────────────────────────────────────┘
                   │
                   │
┌─────────────────────────────────────┐
│                                      │
│         CALCULATE A JOINT ANGLE      │ ──830
│                                      │
└─────────────────────────────────────┘
                   │
                   │
              ◇─────────◇
             ╱           ╲
            ╱     IS      ╲  ──840
           ╱ EITHER OF THE ╲
           ╲    SENSORS    ╱
            ╲   UNWORN?   ╱
             ╲           ╱
              ◇─────────◇
                   │
                   ▼
```

# FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019038225 A1

- WO 2019112158 A1

**Non-patent literature cited in the description**

- **MADGWICK.** Estimation of IMU and MARG orientation using a gradient descent algorithm. *IEEE International Conference on Rehabilitation Robotics,* 2011